# EUROPEAN PATENT APPLICATION

(11) **EP 3 572 027 A1**
(43) Date of publication of application: **27.11.2019**
(21) Application number: 18197519.4
(22) Date of filing: 28.09.2018
(51) Int. Cl.: A61B 34/30, A61B 90/50, A61B 90/10, B25J 18/00, B25J 17/00, A61B 90/00

(54) **ROBOT HAVING STEREOTACTIC FUNCTION**

(30) Priority: 21.05.2018 CN 201820762300 U
(71) Applicant: Horizon Microport Medical Technology (Beijing) Co., Ltd, 100192 Beijing (CN)
(72) Inventor: WANG, Rongjun, Beijing, Beijing 100192 (CN); ZHANG, Jing, Beijing, Beijing 100192 (CN); JIA, Jianqiang, Beijing, Beijing 100192 (CN)
(74) Representative: ZHAOffice SPRL

(57) **Abstract**

The invention relates to a robot having a stereotactic function, belonging to the technical field of medical robots. The robot includes a body, the body has a working face thereon, a mechanical arm is provided on the working face, the mechanical arm is fixedly connected with a guiding means at one end away from the body, the mechanical arm has a first joint component and a second joint component, the first joint component is connected to the working face, the second joint component includes a second joint seat, a second joint shaft, and a second joint arm, an encoder and a clutch are provided on the second joint seat, the clutch controls the second joint shaft to rotate, the second joint shaft is sleeved with a torsion spring thereon assisting the second joint arm to rotate. In this technical solution, with the design of the torsion spring in the second joint component, when an operator rotates the second joint arm to adjust a spatial posture of the mechanical arm, the torsion spring can make a doctor's operation to be more laborsaving, thus reducing a load of the doctor in an operation process.

## Description

### Technical Field

The invention relates to a robot having a stereotactic function, belonging to the technical field of medical robots.

### Background

Surgeries such as minimally invasive treatments for cerebral hemorrhage, ventricular puncture and drainage, brain tissue biopsy and so on are widely used in the current medical field. These checks and treatments require craniocerebral drilling, and a drilling point before a skull is drilled through is generally determined under CT or upon conversion from a CT photograph to a head of a patient. Currently, in the medical field, most of craniocerebral surgeries mainly rely on brain stereotaxic instrument having a frame, but a frame structure has following problems: infants are not accustomed to a fixed frame, a surgery path is relatively deep in the important structural positions in the deep brain, and a shape and a target point of a lesion with a relatively small volume is not displayed clearly, and a positioning accuracy is not good enough. Thus, a brain stereotaxic device without a frame structure is designed, to combine a surgery planning system, a navigation system and a surgery platform together, which is of important significance to improve safety of neurosurgical stereotactic surgeries.

### Summary

In order to solve the problems existing in the prior art, the invention provides a robot having a stereotactic function, which, with improvements on structures, solves the problems of complex operations and poor accuracy of existing devices. A specific technical solution is as follows:
A robot having a stereotactic function, including a body, wherein the body has a working face thereon, a mechanical arm is provided on the working face, the mechanical arm is fixedly connected with a guiding means at one end away from the body, the mechanical arm has a first joint component and a second joint component, the first joint component is connected to the working face, the second joint component includes a second joint seat, a second joint shaft, and a second joint arm, an encoder and a clutch are provided on the second joint seat, the clutch controls the second joint shaft to rotate, the second joint shaft is sleeved with a torsion spring thereon assisting the second joint arm to rotate.

As improvement on the above technical solution, preferably a rotation groove is formed on the second joint seat, a strip-shape notch is provided in a bottom portion of the rotation groove, and a limit block for positioning the second joint arm is provided inside the strip-shape notch.

As improvement on the above technical solution, preferably a positioning block corresponding to the limit block is provided on the second joint arm.

As improvement on the above technical solution, preferably a third joint component, a fourth joint component, and a fifth joint component are further provided between the second joint component of the mechanical arm and the guiding means.

As improvement on the above technical solution, preferably a protection shell is sleeved outside the mechanical arm.

As improvement on the above technical solution, preferably a base plate is provided on the first joint component, and a support block is provided between the base plate and the working face.

As improvement on the above technical solution, preferably a display is provided on the body.

As improvement on the above technical solution, preferably a plurality of handles are provided on both sides of the body, and a plurality of rollers are provided in a bottom portion of the body.

As improvement on the above technical solution, preferably an access port is provided on a side face of the body.

The invention has the following advantages:
(1) With the design of the torsion spring in the second joint component, when an operator rotates the second joint arm to adjust a spatial posture of the mechanical arm, the torsion spring can make a doctor's operation to be more labor-saving, thus reducing a load of the doctor in an operation process;

Preferred embodiments have one or more of the following advantages:
(2) With the use of the strip-shape notch to restrict the limit block, even if the screw on the limit block loosens, a problem of deviation of a zero position will not occur to the limit block;
(3) After various joints are aligned with zero positions, angles of the various joints of the mechanical arm are quickly adjusted to be overlapped with system-formulated angles, relative positions of the various joints are locked with the power being cut off, which is far less in preparation time and far lower in operation difficulty than commercially available imported products, and has good applicability;
(4) The working face is designed in a waterproof manner, then even if a liquid spills on the working face in the surgery process, it will not leak into the inside of the body to cause damage to internal hardware devices of the robot, thus ensuring smooth conduction of the surgery;
(5) The handles are provided on both sides of the body, the handles at the two sides can realize fixation between a surgery bed of a patient and the robot, thus ensuring that the robot is free of shaking in the surgery process. The body is provided with the rollers in the bottom portion, and the rollers serve the function of assisting movement of the robot. The bottom portion of the body can be provided to be height-adjustable, so as to be matched with the surgery beds of different heights, and improve flexibility of application of the robots.

### Brief Description of the Drawings

FIG. 1 is a schematic structural view of a robot having a stereotactic function according to the invention.
FIG. 2 is a front view of a mechanical arm in the invention.
FIG. 3 is a right view of the mechanical arm in the invention.
FIG. 4 is a schematic view of a position where a torsion spring is provided in a second joint component in the invention.

### Detailed Description

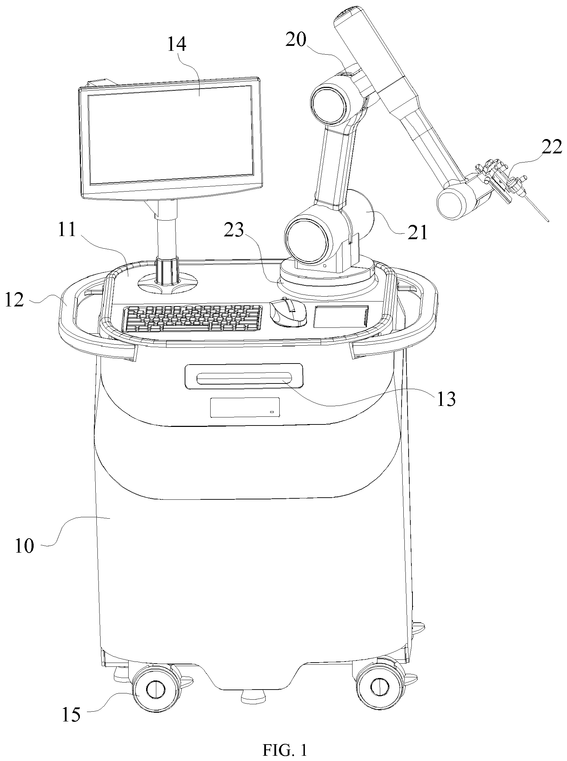

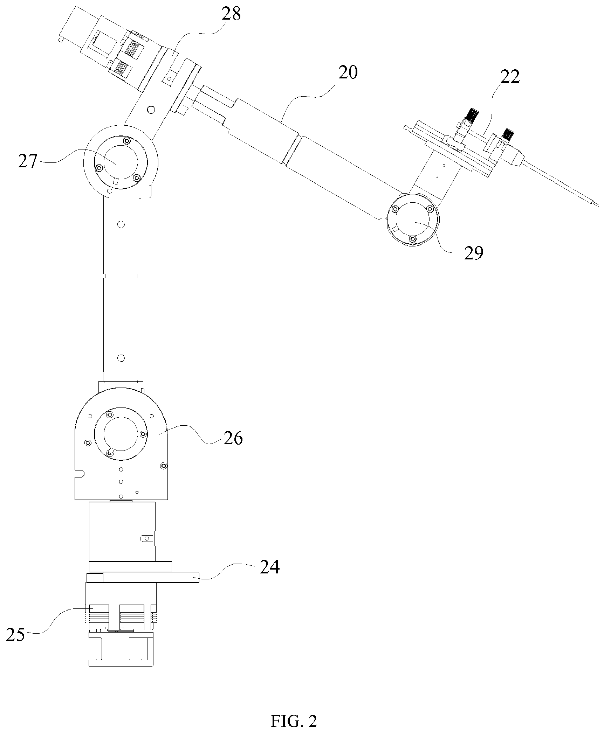

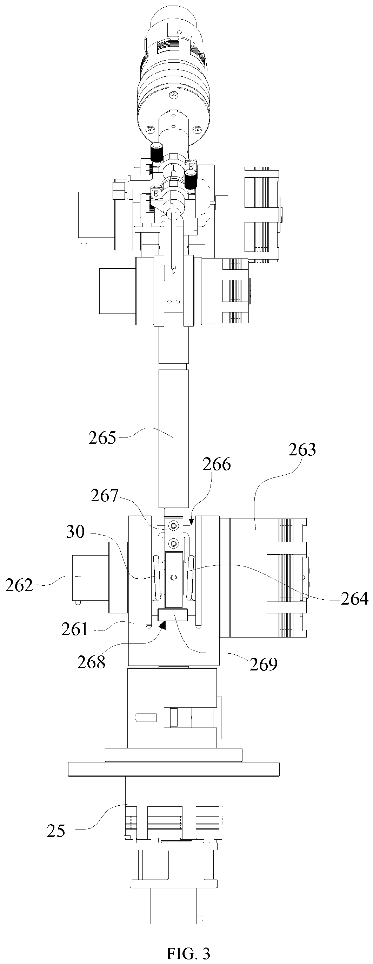

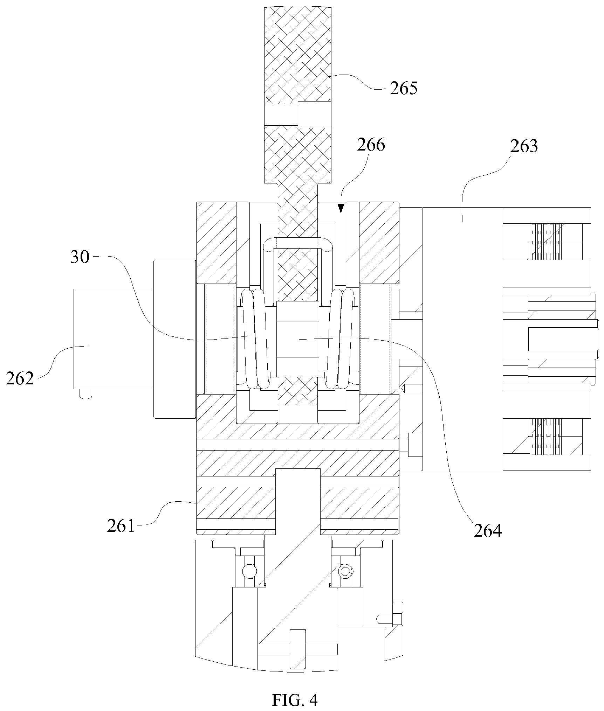

As illustrated in FIG. 1, FIG. 2, FIG. 3, and FIG. 4, the invention provides a robot having a stereotactic function, including a body 10. The body 10 has a working face 11 thereon. A mechanical arm 20 is provided on the working face 11. The mechanical arm 20 is fixedly connected with a guiding means 22 at one end away from the body 10. The mechanical arm 20 has a first joint component 25 and a second joint component 26. The first joint component 25 is connected to the working face 11. The second joint component 26 includes a second joint seat 261, a second joint shaft 264, and a second joint arm 265. An encoder 262 and a clutch 263 are provided on the second joint seat 261. The clutch 263 controls the second joint shaft 264 to rotate. The second joint shaft 264 is sleeved with a torsion spring 30 thereon assisting the second joint arm 265 to rotate.

In this solution, the working face 11 on the body 1 is similar to an operation platform. Externals devices such as a display 14, a control panel, a keyboard, a mouse and so on can be additionally provided on the working face 11. The working face 11 can be designed in a waterproof manner, thus improving the protection to a host machine inside the body 10. The body 10 is provided with an access port 13 on a side face, and the access port 13 can be connected to an external device storing surgery information. The mechanical arm 20 is provided on the working face 11. The mechanical arm 20 includes at least two joint components, for example, the mechanical arm can be structured by five joint components, i.e. the first joint component 25, the second joint component 26, a third joint component 27, a fourth joint component 28, and a fifth joint component 29, and one guiding means 22. Each joint component is provided with an encoder and a clutch, wherein the encoder serves a function of converting an angular displacement of a rotation shaft into an electrical signal, and the clutch is used to control the combination of the rotation shaft, thus realizing control over the rotation shaft. Just through synergistic cooperation of the encoders and the clutches of the multiple joint components, precise positioning of the mechanical arm in a three-dimensional space can be realized. The guiding means 22 is connected to a drill head. After spatial positioning of the mechanical arm is completed, a depth of the drill head is controlled by the guiding means 22. By adjusting various parts of the guiding means 22, it is convenient to realize a distance from a head of a patient, thus reducing a precision problem brought about by an end error.

The second joint seat 261 of the second joint component 26 is connected to the first joint component 25. The second joint component 26 is opened with a rotation groove 266. The second joint shaft 264 traverses the rotation groove 266. The second joint arm 265 is fixedly connected onto the second joint shaft 264, and the second joint arm 265 rotates with the second joint shaft 264. The second joint seat 261 is further provided with the encoder 262 and the clutch 263. The clutch 263 controls the second joint shaft 264 to rotate. The torsion spring 30 is sleeved on the second joint shaft 264. The torsion spring 30 is on one side fixedly connected to the rotation groove 266 of the second joint seat 261, and abuts against the second joint arm 265 on the other side. When an operator rotates the second joint arm 265 to adjust a spatial posture of the mechanical arm, the torsion spring 30 can enable a doctor's operation to be more labor-saving, thus reducing a load of the doctor in an operation process. A reason of providing the torsion spring 30 at the second joint component 26 is that the joint component at this place bears most of a dead weight of the mechanical arm, then after the torsion spring 30 is added, a rotation process of this joint component is smooth, which is of important significance to improve and adjust the precision of spatial positioning and increase the service life of the device.

Furthermore, the rotation groove 266 is opened with a strip-shape notch 268 in a bottom portion, and a limit block 269 for positioning the second joint arm 265 is provided inside the strip-shape notch 268. A conventional limit block is fixedly connected to a joint seat merely through a screw, then once the screw loosens, the limit block will loosen, thus a joint arm cannot be precisely positioned to a zero initial position. With the use of the design of such strip-shape notch 268, the limit block 269 can be restricted by the notch, then even if the screw on the limit block 269 loosens, a problem of deviation of a zero position will not occur to the limit block 269.

Furthermore, a positioning block 267 corresponding to the limit block 269 is provided on the second joint arm 265, and the positioning block 267 is corresponding to the limit block 269.

The first joint component 25 is provided with a base plate 24. A support block 23 is provided between the base plate 24 and the working face 11. The support block 23 can improve reliability of connection between the mechanical arm and the working face 11. Various joint components of the mechanical arm 20 are sleeved with a protection shell 21 outside. Since there are relatively many structures inside the mechanical arm 20, and a high degree of precision is demanded, the protection shells 21 can protect the mechanical arm 20 in the surgery process, thus improving the service life of the mechanical arm 20.

The body 10 is provided with handles 12 at two sides. The handles 12 at the two sides can realize fixation between a surgery bed of a patient and the robot, thus ensuring that the robot is free of shaking in a surgery process. The body 10 is provided with rollers 15 in a bottom portion, and the rollers 15 serve a function of assisting movement of the robot. The bottom portion of the body 10 can be provided to be height-adjustable, so as to be matched with the surgery beds of different heights.

Although the invention is described in detail in the above through general description and specific embodiments, on the basis of the invention, some modifications or improvements can be made thereto, which is apparent to a person skilled in the art. Therefore, these modifications or improvements made without departing from the spirit of the invention fall within the scope of protection claimed in the invention.

## Claims

1. A robot having a stereotactic function, **characterized by** comprising a body, wherein the body has a working face thereon, a mechanical arm is provided on the working face, the mechanical arm is fixedly connected with a guiding means at one end away from the body, the mechanical arm has a first joint component and a second joint component, the first joint component is connected to the working face, the second joint component comprises a second joint seat, a second joint shaft, and a second joint arm, an encoder and a clutch are provided on the second joint seat, the clutch controls the second joint shaft to rotate, the second joint shaft is sleeved with a torsion spring thereon assisting the second joint arm to rotate.

2. The robot having a stereotactic function of claim 1, **characterized in that** a rotation groove is formed on the second joint seat, a strip-shape notch is provided in a bottom portion of the rotation groove, and a limit block for positioning the second joint arm is provided inside the strip-shape notch.

3. The robot having a stereotactic function of claim 1, 2 or 3, **characterized in that** a positioning block corresponding to the limit block is provided on the second joint arm.

4. The robot having a stereotactic function of any of the preceding claims, **characterized in that** a third joint component, a fourth joint component, and a fifth joint component are further provided between the second joint component of the mechanical arm and the guiding means.

5. The robot having a stereotactic function of any of the preceding claims, **characterized in that** a protection shell is sleeved outside the mechanical arm.

6. The robot having a stereotactic function of any of the preceding claims, **characterized in that** a base plate is provided on the first joint component, and a support block is provided between the base plate and the working face.

7. The robot having a stereotactic function of any of the preceding claims, **characterized in that** a display is provided on the body.

8. The robot having a stereotactic function of any of the preceding claims, **characterized in that** a plurality of handles are provided on both sides of the body, and a plurality of rollers are provided in a bottom portion of the body.

9. The robot having a stereotactic function of any of the preceding claims, **characterized in that** an access port is provided on a side face of the body.
